# EUROPEAN PATENT APPLICATION

(11) **EP 1 627 659 A1**
(43) Date of publication of application: **22.02.2006**
(21) Application number: 05255026.6
(22) Date of filing: 15.08.2005
(51) Int. Cl.: A61N 1/36, A61N 1/05

(54) **Electrical stimulation system and method for stimulating nerve tissue in the brain using a stimulation lead having a tip electrode, having at least five electrodes, or both**

(30) Priority: 17.08.2004 US 602136 P
(71) Applicant: ADVANCED NEUROMODULATION SYSTEMS, INC., Plano, TX 75024 (US)
(72) Inventor: Cameron, Tracy L., Toronto Ontario M6P 3H1 (CA); Black, Damon R., Dallas Texas 75223 (US); Jones, Timothy S., Carrollton Texas 75007 (US)
(74) Representative: Lawrence, John

(57) **Abstract**

An electrical stimulation system (10) is provided for electrically stimulating target nerve tissue in a person's brain using a stimulation lead (14) having a tip electrode (18a), having at least five electrodes (18), or both. The system (10) includes a stimulation lead (14) adapted for implantation into the brain for electrical stimulation of target nerve tissue in the brain. The stimulation lead (14) may include a tip electrode (18a) located at a distal tip (19) of the stimulation lead (14) and adapted to be positioned proximate the target nerve tissue in the brain and to deliver electrical stimulation pulses to the target nerve tissue in the brain. The stimulation lead (14) may include at least five electrodes (18), one or more of which are adapted to be positioned proximate the target nerve tissue in the brain and to deliver electrical stimulation pulses to the target nerve tissue in the brain. The system (10) also includes a stimulation source (12) operable to generate the electrical stimulation pulses for transmission to the one or more electrodes (18), possibly including a tip electrode (18a), of the stimulation lead (14) to cause the one or more electrodes (18) to deliver the electrical stimulation pulses to the target nerve tissue in the brain.

To be accompanied, when published, with Figure 1a of the drawings.

## Description

### RELATED APPLICATION

This application claims priority under 35 U.S.C. §119 of provisional application serial number 60/602,136, filed August 17, 2004.

### TECHNICAL FIELD OF THE INVENTION

This invention relates generally to electrical stimulation of a person's brain and in particular to an electrical stimulation system and method for stimulating nerve tissue in the brain using a stimulation lead having a tip electrode, having at least five electrodes, or both.

### BACKGROUND

Many people experience adverse conditions associated with functions of the cortex, the thalamus, and other deep brain structures. Such conditions have been treated effectively by delivering electrical energy to one or more target areas of the deep brain. One method of delivering electrical energy to the brain involves inserting a stimulation lead through a burr hole formed in the skull and then positioning the stimulation lead in a precise location proximate a target area of the brain to be stimulated such that stimulation of the target area causes a desired clinical effect. For example, one desired clinical effect may be cessation of tremor from a movement disorder such as Parkinson's Disease. A variety of other clinical conditions may also be treated with deep brain stimulation (DBS), such as essential tremor, tremor from multiple sclerosis or brain injury, or dystonia or other movement disorders. The stimulation lead implanted in the brain is connected to a stimulation pulse generator implanted at a separate site in the body, such as in the chest or buttocks.

Stimulation leads used in DBS typically include four conventionally-shaped circumferential electrodes spaced apart from the distal end of the stimulation lead and from one another along the stimulation lead that cooperate to stimulate a target area of the brain. Because of the small size of the target area and the close proximity of non-targeted areas, stimulation leads implanted in the brain require precise placement in the brain. To properly position an electrode proximate a target area, however, the distal tip of the stimulation lead often must be inserted further into the brain than desired, possibly into non-targeted areas of the brain. Accordingly, stimulation leads used in DBS may result in unnecessary damage or risk of damage to non-targeted areas. In addition, conventionally-shaped circumferential electrodes of stimulation leads used in DBS deliver stimulation energy radially in all directions, resulting in a stimulation field that may be less desirable than can be generated using other electrode geometries.

### SUMMARY OF THE INVENTION

The electrical stimulation system and method of the present invention may reduce or eliminate certain problems and disadvantages associated with previous techniques for stimulating nerve tissue in the brain.

According to one embodiment, an electrical stimulation system is provided for electrically stimulating target nerve tissue in a person's brain using a stimulation lead having a tip electrode. The system includes a stimulation lead adapted for implantation into the brain for electrical stimulation of target nerve tissue in the brain. The stimulation lead includes a tip electrode located at a distal tip of the stimulation lead and adapted to be positioned proximate the target nerve tissue in the brain and to deliver electrical stimulation pulses to the target nerve tissue in the brain. The system also includes a stimulation source operable to generate the electrical stimulation pulses for transmission to the tip electrode located at the distal tip of the stimulation lead to cause the tip electrode to deliver the electrical stimulation pulses to the target nerve tissue in the brain.

According to another embodiment, an electrical stimulation system is provided for electrically stimulating target nerve tissue in a person's brain using a stimulation lead having at least five electrodes. The system includes a stimulation lead adapted for implantation into the brain for electrical stimulation of target nerve tissue in the brain. The stimulation lead includes at least five electrodes. The stimulation lead is adapted to be implanted in the brain such that one or more of the at least five electrodes are positioned proximate the target nerve tissue in the brain to deliver electrical stimulation pulses to the target nerve tissue in the brain. The system also includes a stimulation source operable to generate the electrical stimulation pulses for transmission to the one or more of the at least five electrodes of the stimulation lead positioned proximate the target nerve tissue in the brain to cause the one or more of the at least five electrodes to deliver the electrical stimulation pulses to the target nerve tissue in the brain.

Particular embodiments of the present invention may provide one or more technical advantages. According to the present invention, an electrical stimulation system is used to provide therapeutic electrical stimulation of target nerve tissue in a person's brain using a stimulation lead having a tip electrode, having at least five electrodes, or both. In certain embodiments, a stimulation lead having one or more electrodes including a tip electrode may be implanted in the brain such that at least the tip electrode is located proximate target nerve tissue in the brain. In certain embodiments, the tip electrode is a substantially spherical electrode that generates a substantially monopolar field that may be more desirable for stimulation of target nerve tissue in the brain than fields generated by circumferential electrodes. In certain embodiments, a stimulation lead having at least five electrodes is implanted in the brain such that one or more of the at least five electrodes are located proximate target nerve tissue in the brain. In certain embodiments, providing five or more electrodes allows additional flexibility and performance compared to stimulation leads having only four electrodes. In certain embodiments, the target nerve tissue in the brain includes deep brain tissue and the stimulation system provides DBS.

Certain embodiments may provide all, some, or none of these advantages. Certain embodiments may provide one or more other advantages, one or more of which may be apparent to those skilled in the art from the figures, descriptions, and claims included herein.

### BRIEF DESCRIPTION OF THE DRAWINGS

For a more complete understanding of the present invention and advantages thereof, reference is now made to the following description taken in conjunction with the accompanying drawings, in which:
**FIGURES 1A-1B** illustrate example stimulation systems for electrically stimulating target nerve tissue in the brain using a stimulation lead having a tip electrode, having at least five electrodes, or both.
**FIGURES 2A-2D** illustrate example stimulation leads, each having a tip electrode, at least five electrodes, or both, that may be used to electrically stimulate target nerve tissue in the brain;
**FIGURE 3** illustrates example implantation of a stimulation lead into a person's brain;
**FIGURE 4** illustrates an example method for implanting the stimulation system of FIGURES 1A-1B into a person to electrically stimulate target nerve tissue in the brain;
**FIGURE 5** illustrates an example stimulation set;
**FIGURE 6** illustrates a number of example stimulation programs, each of which includes a number of stimulation sets; and
**FIGURE 7** illustrates example execution of a sequence of stimulation sets within an example stimulation program.

### DESCRIPTION OF EXAMPLE EMBODIMENTS

According to the present invention, an electrical stimulation system is used to electrically stimulate target nerve tissue in a person's brain using a stimulation lead having a tip electrode, having at least five electrodes, or both. As used herein, the term "proximate" means on, in, adjacent, or near. A stimulation lead according to the present invention may, but need not, have both a tip electrode and at least five electrodes. In certain embodiments, a stimulation lead having one or more electrodes including a tip electrode is implanted in a person's brain such that at least the tip electrode is located proximate the target nerve tissue in the brain. Thus, in these embodiments, at least the tip electrode of the stimulation lead is adapted to be positioned on, in, adjacent, or near the target nerve tissue in the brain. In certain embodiments, a stimulation lead having at least five electrodes is implanted in a person's brain such that one or more of the at least five electrodes are located proximate the target nerve tissue in the brain. Thus, in these embodiments, the one or more of the at least five electrodes are adapted to be positioned on, in, adjacent, or near the target nerve tissue in the brain. In addition, the term "nerve tissue in the brain" as used herein includes any neural tissue in any neural region of the brain, including gray matter and white matter that make up the brain. In certain embodiments, "nerve tissue in the brain" may include deep brain tissue, and the stimulation system may be used for DBS. The stimulation lead may be precisely positioned using a neuronavigation system that includes brain imaging and mapping data received from imaging of the person's brain or of the brains of other patients. In operation, one or more selected electrodes of the stimulation lead deliver electrical stimulation pulses to the target nerve tissue in the brain for therapeutic purposes.

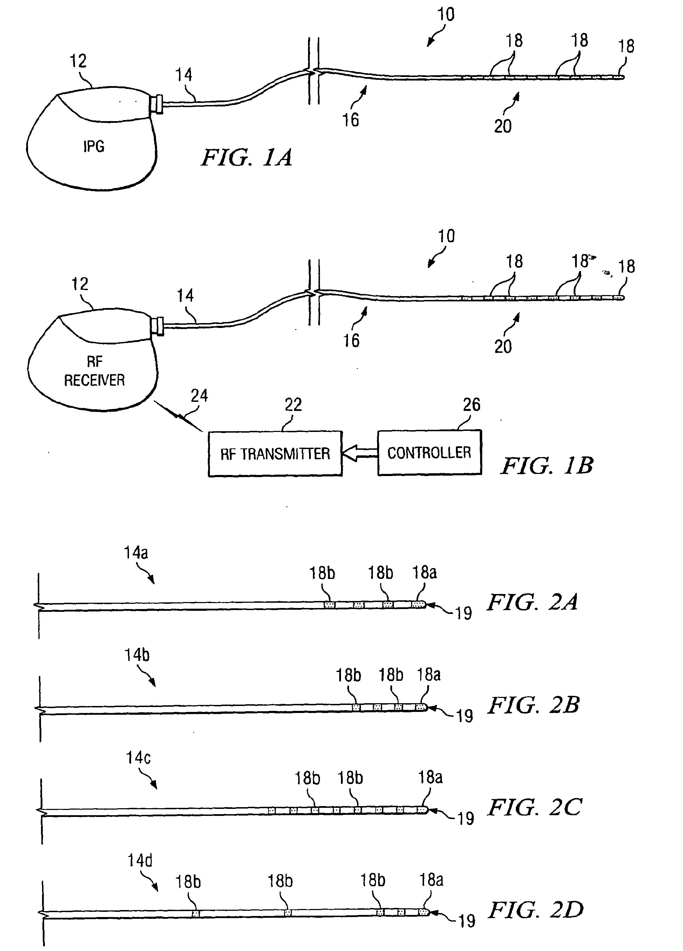

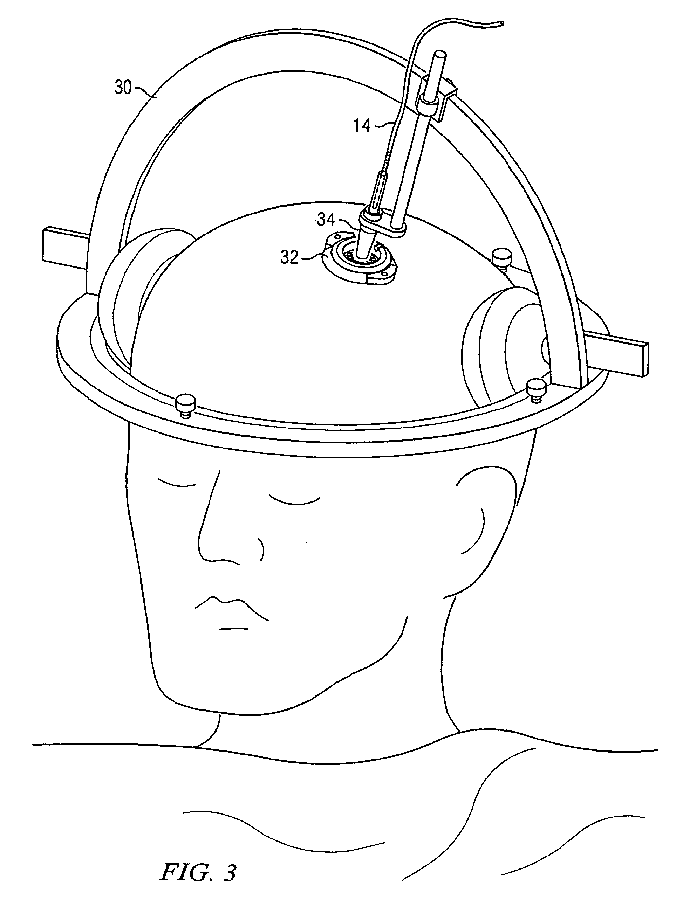

FIGURES 1A-1B illustrate example electrical stimulation systems 10 for electrically stimulating target nerve tissue in the brain using a stimulation lead having a tip electrode, having at least five electrodes, or both. In general terms, stimulation system 10 includes an implantable electrical stimulation source 12 and an implantable electrical stimulation lead 14 for applying electrical stimulation pulses to the target nerve tissue. In operation, both of these primary components are implanted in the person's body. In particular, stimulation source 12 may be implanted within a subcutaneous pocket within the person's torso (such as in or near the chest or buttocks). However, stimulation source 12 may be located at any suitable location within the person's body according to particular needs. A connecting portion 16 is tunneled, at least in part, subcutaneously underneath the person's skin to connect stimulation source 12 with stimulation lead 14. Stimulation lead 14 may be implanted within the person's brain as discussed below with reference to FIGURE 3. Stimulation source 12 controls the stimulation pulses transmitted to one or more electrodes 18, in certain embodiments including at least a tip electrode 18, located on a stimulating portion 20 of stimulation lead 14 according to suitable stimulation parameters (e.g., duration, amplitude or intensity, frequency, etc.). A doctor, the patient, or another user of stimulation source 12 may directly or indirectly input stimulation parameters to specify or modify the nature of the electrical stimulation provided.

In one embodiment, as shown in FIGURE 1A, stimulation source 12 includes an implantable pulse generator (IPG). An example IPG may be one manufactured by Advanced Neuromodulation Systems, Inc., such as the Genesis® System, part numbers 3604, 3608, 3609, and 3644. In another embodiment, as shown in FIGURE 1B, stimulation source 12 includes an implantable wireless receiver. An example wireless receiver may be one manufactured by Advanced Neuromodulation Systems, Inc., such as the Renew® System, part numbers 3408 and 3416. The wireless receiver is capable of receiving wireless signals from a wireless transmitter 22 located external to the person's body. The wireless signals are represented in FIGURE 1B by wireless link symbol 24. A doctor, the patient, or another user of stimulation source 12 may use a controller 26 located external to the person's body to provide control signals for operation of stimulation source 12. Controller 26 provides the control signals to wireless transmitter 22, wireless transmitter 22 transmits the control signals and power to the wireless receiver of stimulation source 12, and stimulation source 12 uses the control signals to vary the stimulation parameters of electrical stimulation pulses transmitted through stimulation lead 14 to the stimulation site. An example wireless transmitter 122 may be one manufactured by Advanced Neuromodulation Systems, Inc., such as the Renew® System, part numbers 3508 and 3516.

FIGURES 2A-2D illustrate example electrical stimulation leads 14 that may be used for electrically stimulating target nerve tissue in the brain. Each example stimulation lead 14 has a tip electrode 18, at least five electrodes 18, or both. Stimulation leads 14 are preferably percutaneous leads.

As described above, in certain embodiments, stimulation lead 14 incorporated in stimulation system 10 includes at least a tip electrode 18a located at a distal tip 19 of stimulation lead 14. Tip electrode 18a is adapted to be positioned proximate the target nerve tissue in the brain and used to deliver to the target nerve tissue stimulation pulses received from stimulation source 12. Tip electrode 18a may include platinum, iridium, another suitable metal, or any suitable combination thereof. In certain embodiments, tip electrode 18a has a diameter of approximately 1.40 mm. Tip electrode 18a preferably has a substantially spherical or dome-shaped distal end. A portion of tip electrode 18a coupled to its substantially spherical distal end may be configured similar to and operate similar to an analogous portion of a conventionally-shaped circumferential electrode 18. In embodiments in which tip electrode 18a has a substantially spherical distal end, at least the distal end of tip electrode 18a may provide a substantially monopolar field, emitting electrical stimulation energy in substantially all directions to the target nerve tissue in the brain. Alternatively, tip electrode 18a may be, in its entirety, a conventionally-shaped electrode located at distal tip 19 of stimulation lead 14. In embodiments in which tip electrode 18a is a conventionally-shaped circumferential electrode, tip electrode 18a may emit electrical stimulation energy generally radially (i.e. generally perpendicular to the axis of tip electrode 18a) in all directions. In certain embodiments, such as where stimulation lead 14 includes only tip electrode 18a, tip electrode 18a may act as a cathode and a terminal or other contact associated with stimulation source 12 may act as an anode.

As described above, in certain embodiments, stimulation lead 14 incorporated in stimulation system 10 includes at least five electrodes 18 spaced apart from one another along stimulation lead 14. Preferably, the at least five electrodes 18 include a tip electrode 18a having a substantially spherical or dome-shaped distal end as described above and the other electrodes 18 may be conventionally-shaped circumferential electrodes 18b spaced apart from tip electrode 18a and from one another along stimulating portion 20 of stimulation lead 14. Alternatively, the at least five electrodes 18 may all be conventionally-shaped circumferential electrodes 18. Such circumferential electrodes 18 may be conventionally placed, the most distal electrode 18 being separated from the distal end of stimulation lead 14, or may be placed such that the most distal electrode 18 is a tip electrode 18a located at the distal end of stimulation lead 14. Circumferential electrodes 18 may emit electrical stimulation energy generally radially (i.e. generally perpendicular to the axis of electrode 18) in all directions. Circumferential electrodes 18b may be positioned such that they deliver electrical stimulation pulses to the same or different target nerve tissue as any tip electrode 18a.

Although various types of stimulation leads 14 are shown as examples, the present invention contemplates stimulation system 10 including any suitable type of stimulation lead 14 in any suitable number. In addition, a stimulation lead 14 may be used alone or in combination with one or more other stimulation leads 14. For example, unilateral stimulation of nerve tissue in the brain is typically accomplished using a single stimulation lead 14 implanted in one side of the brain, while bilateral stimulation of the brain is typically accomplished using two stimulation leads 14 implanted in opposite sides of the brain.

FIGURE 3 illustrates an example of a person undergoing implantation of a stimulation lead 14, for DBS for example, using stereotactic equipment 30 to guide lead placement and a burr hole cap or other apparatus 32 to secure stimulation lead 14 in position in the person's brain. As can be appreciated from FIGURE 3, stimulation lead 14 is typically coupled to stereotactic equipment 30 during lead placement for increased stability and housed within an insertion cannula 34 for insertion into the brain.

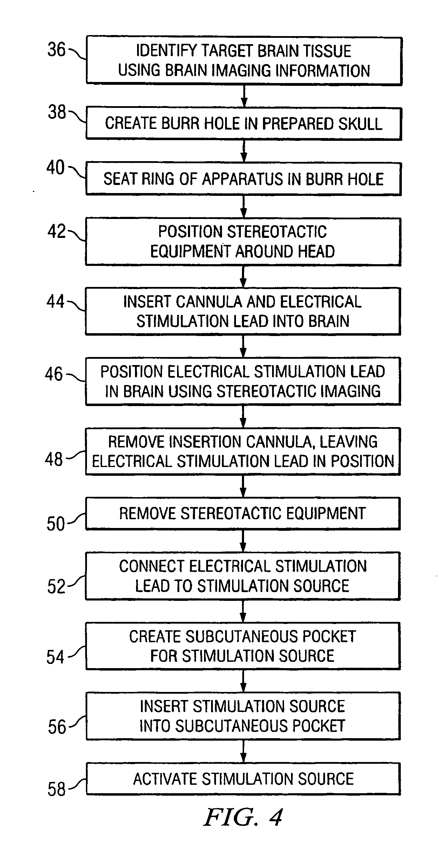

FIGURE 4 illustrates an example method for electrically stimulating target nerve tissue in the brain using stimulation system 10 of FIGURES 1A-1B. At step 36, at least a portion of the person's brain may be imaged or mapped using one or more imaging techniques to obtain brain imaging information and to identify target nerve tissue in the brain. Those in the art will understand, however, that the location of the target nerve tissue in the person's brain may be determined using information from brain imaging studies performed on other patients, and thus the imaging of the person's brain at step 36 may not be performed.

At steps 38 through 50, stimulation lead 14 is implanted into the brain, for DBS for example. The skull is first prepared by exposing the skull and creating a burr hole in the skull at step 38. Apparatus 32 is inserted into the burr hole and may, but not need, be rotated within the burr hole so that apparatus 32 is properly aligned with connecting portion 16 of stimulation lead 14 when connecting portion 16 is subsequently laid substantially flat along the skull. Apparatus 32 is then fixed to the scalp or skull using sutures, screws, or other suitable fixators at step 40. At step 42, stereotactic equipment 30 suitable to aid in placement of stimulation lead 14 in the brain may be positioned around the head. Insertion cannula 34 for stimulation lead 14 is inserted into the brain at step 44. For example, a hollow needle may provide cannula 34. Cannula 34 and stimulation lead 14 may be inserted together or stimulation lead 14 may be inserted through cannula 34 after cannula 34 has been inserted. Using stereotactic imaging guidance or otherwise, stimulation lead 14 is then precisely positioned such that one or more electrodes 18, in certain embodiments including at least tip electrode 18a, are located proximate the target nerve tissue in the brain at step 46. The target nerve tissue may include deep brain tissue for DBS.

Once stimulation lead 14 has been positioned in the brain, stimulation lead 14 is uncoupled from any stereotactic equipment 30, and cannula 34 and any stereotactic equipment 30 are removed at steps 48 and 50. Where stereotactic equipment 30 is used, cannula 34 may be removed before, during, or after removal of stereotactic equipment 30. Connecting portion 16 of electrical stimulation lead 14 is secured using apparatus 32 and laid substantially flat along the skull.

At step 52, stimulation lead 14 is connected to stimulation source 12, which may also be implanted in the person's body. Implantation of stimulation source 12 may include creating a subcutaneous pocket formed to receive and house stimulation source 12 at step 54. The implant site is usually positioned a distance away from the insertion site, such as in or near the chest or buttocks. Once all appropriate components of stimulation system 10 are implanted, stimulation source 12 may be activated at step 58. Activation of stimulation source 12 generates and delivers electrical stimulation pulses via stimulation lead 14 to the target nerve tissue proximate one or more electrodes 18, in certain embodiments including at least tip electrode 18a, of stimulation lead 14.

Although example steps are illustrated and described, the present invention contemplates two or more steps taking place substantially simultaneously or in a different order. In addition, the present invention contemplates using methods with additional steps, fewer steps, or different steps, so long as the steps remain appropriate for implanting stimulation system 10 into a person for electrical stimulation of the person's brain using a stimulation lead having a tip electrode 18a, having at least five electrodes 18, or both.

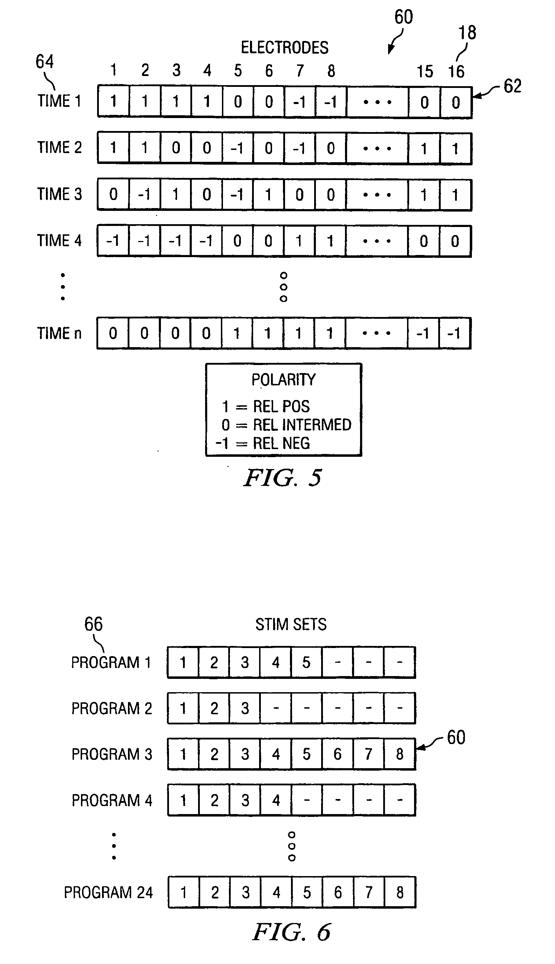

FIGURE 5 illustrates an example stimulation set 60. One or more stimulation sets 60 may be provided, each stimulation set 60 specifying a number of stimulation parameters for the stimulation set 60. For example, as described more fully below with reference to FIGURES 6-7, multiple stimulation sets 60 may be executed in an appropriate sequence according to a pre-programmed or randomized stimulation program. Stimulation parameters for a stimulation set 60 may include an amplitude or intensity, a frequency, phase information, and a pulse width for each of a series of stimulation pulses that electrodes 18, in certain embodiments including at least tip electrode 18a, are to deliver to the target nerve tissue in the brain during a time interval during which stimulation set 60 is executed, along with a polarity 62 for each electrode 18 within each stimulation pulse. In general, electric fields are generated between two adjacent or other electrodes 18 having different polarities 62 to deliver electrical stimulation pulses to target nerve tissue in the brain. One of these electrodes 18 acts as a cathode and the other of the electrodes 18 acts as an anode. In certain embodiments, such as where stimulation lead 14 includes only a tip electrode 18a, tip electrode 18a may act as a cathode and a terminal or other contact associated with stimulation source 12 may act as an anode. Stimulation parameters may also include a pulse shape, for example, biphasic cathode first, biphasic anode first, or any other suitable pulse shape.

During the operation of stimulation system 10 according to a particular set of stimulation parameters, the efficacy of the stimulation associated with the particular set of stimulation parameters may decrease over time due to neuroplasticity of the brain. Neuroplasticity refers to the ability of the brain to dynamically reorganize itself in response to certain stimuli to form new neural connections. This allows the neurons in the brain to compensate for injury or disease and adjust their activity in response to new situations or changes in their environment. With respect to electrical stimulation, the reduction in efficacy due to neuroplasticity can occur after just a few weeks of treatment. In order to regain the same efficacy, a new set of efficacious stimulation parameters must be determined, the new set of stimulation parameters must be entered into the system, and the system is again used to electrically stimulate the brain according to the new set of stimulation parameters to continue to treat the condition. This may result in the additional time and expense associated with a return visit to the treating physician for determining and entering the new set of stimulation parameters. Especially where treatment is to continue over a relatively long period of time, such as months or years, this additional time and expense poses a significant drawback.

Thus, in certain embodiments, in addition to providing therapeutic electrical stimulation to the brain for treating the condition in the person's body, stimulation system 10 may be capable of applying additional electrical stimulation to the brain to reduce neuroplasticity effects associated with the therapeutic electrical stimulation. In one embodiment, the nature of the neuroplasticity reducing electrical stimulation may be varied more or less continually, in a predetermined or randomized manner, to prevent, delay, or otherwise reduce the ability of the brain to adapt to the neuroplasticity reducing electrical stimulation and dynamically reorganize itself accordingly. In a more particular embodiment, the neuroplasticity reducing electrical stimulation may be randomized or otherwise varied about the therapeutic electrical stimulation to achieve this result. In essence, the randomized or otherwise varied neuroplasticity reducing electrical stimulation makes it more difficult for the brain to dynamically reorganize itself to overcome the effects of the therapeutic electrical stimulation.

For reducing neuroplasticity effects associated with therapeutic electrical stimulation, one or more stimulation parameters for a stimulation set 60 may be randomized or otherwise varied in any suitable manner within the time interval in which stimulation set 60 is executed, spanning one or more stimulation pulses within each stimulation pulse. For example, instead of or in addition to randomizing or otherwise varying polarities 62 for one or more electrodes 18, in certain embodiments including at least tip electrode 18a, as described below, the amplitude or intensity, frequency, phase information, and pulse width may be randomized or otherwise varied within predetermined ranges, singly or in any suitable combination, within each stimulation pulse. As another example, instead of or in addition to randomizing or otherwise varying polarities 62 for one or more electrodes 18, in certain embodiments including at least tip electrode 18a, over multiple stimulation pulses as described more fully below, the amplitude or intensity, frequency, phase information, and pulse width may be randomized or otherwise varied within predetermined ranges, singly or in any suitable combination, over multiple stimulation pulses, where the combination of stimulation parameters is substantially constant within each stimulation pulse but different for successive stimulation pulses. Such randomization or other variation of stimulation parameters for a stimulation set 60 reduces the ability of the brain to adapt to the neuroplasticity reducing electrical stimulation and dynamically reorganize itself to overcome the effects of the neuroplasticity reducing stimulation.

In certain other embodiments, stimulation system 10 may similarly be capable of applying additional electrical stimulation to the brain to enhance, rather than reduce, neuroplasticity effects associated with the therapeutic electrical stimulation. In one embodiment, the nature of the neuroplasticity enhancing electrical stimulation may be controlled in a predetermined non-randomized manner to promote, accelerate, or otherwise enhance the ability of the brain to adapt to the neuroplasticity enhancing electrical stimulation and dynamically reorganize itself accordingly. In essence, the predetermined non-randomized neuroplasticity enhancing electrical stimulation facilitates the brain dynamically reorganizing itself in response to the therapeutic electrical stimulation. It should be understood that techniques analogous to some or all of those discussed below for reducing neuroplasticity effects may be employed for enhancing neuroplasticity effects.

The polarity for an electrode 18, in certain embodiments including tip electrode 18a, at a time 64 beginning a corresponding stimulation pulse or sub-interval within a stimulation pulse may be a relatively positive polarity 62, a relatively negative polarity 62, or an intermediate polarity 62 between the relatively positive polarity 62 and relatively negative polarity 62. For example, the relatively positive polarity 62 may involve a positive voltage, the relatively negative polarity 62 may involve a negative voltage, and the relatively intermediate polarity 62 may involve a zero voltage (i.e. "high impedance"). In certain embodiments, a zero voltage may correspond to the electrode 18 being "turned off." As another example, the relatively positive polarity 62 may involve a first negative voltage, the relatively negative polarity 62 may involve a second negative voltage more negative than the first negative voltage, and the relatively intermediate polarity 62 may involve a negative voltage between the first and second negative voltages. The availability of three distinct polarities 62 for an electrode 18 may be referred to as "tri-state" electrode operation. The polarity 62 for each electrode 18, in certain embodiments including tip electrode 18a, may change for each of the sequence of times 64 corresponding to stimulation pulses or to sub-intervals within a stimulation pulse according to the stimulation parameters specified for the stimulation set 60. For example, as is illustrated in FIGURE 5 for an example stimulation set 60 for a stimulation lead 14 with sixteen electrodes 18, in certain embodiments including tip electrode 18a, the polarities 62 of the sixteen electrodes 18 may change for each of the sequence of times 64. In the example of FIGURE 5, a relatively positive polarity 62 is represented using a "1," a relatively intermediate polarity 62 is represented using a "0," and a relatively negative polarity 62 is represented using a "-1," although any suitable values or other representations may be used.

Where appropriate, the polarity 62 for each electrode 18, in certain embodiments including tip electrode 18a, may change in a predetermined or randomized manner, randomized changes possibly being more effective with respect to any neuroplasticity reducing stimulation for reasons described above.

Where stimulation system 10 provides, in addition to therapeutic electrical stimulation, electrical stimulation to reduce neuroplasticity effects associated with the therapeutic electrical stimulation, each stimulation pulse or sub-interval within a stimulation pulse may be particular to the stimulation being provided; that is, either to therapeutic electrical stimulation or to neuroplasticity reducing electrical stimulation. For example, one or more stimulation pulses or sub-intervals may be designed to provide therapeutic electrical stimulation and one or more other stimulation pulses or sub-intervals may be designed to reduce neuroplasticity effects. In this case, the therapeutic stimulation pulses or sub-intervals and neuroplasticity reducing stimulation pulses or sub-intervals may be arranged temporally in any suitable manner. A therapeutic stimulation pulse or sub-interval may be separated from a successive therapeutic stimulation pulse or sub-interval by any number of neuroplasticity reducing stimulation pulses or sub-intervals and this number may be the same between each pair of therapeutic stimulation pulses or sub-intervals or may vary between each pair of therapeutic stimulation pulses or sub-intervals in a predetermined or randomized manner. As another example, one or more stimulation pulses or sub-intervals may be designed to concurrently provide both therapeutic and neuroplasticity reducing electrical stimulation.

Similarly, where stimulation system 10 provides, in addition to therapeutic electrical stimulation, electrical stimulation to reduce neuroplasticity effects associated with the therapeutic electrical stimulation, each stimulation set 60 may be particular to either the therapeutic electrical stimulation or the neuroplasticity reducing electrical stimulation. For example, one or more stimulation sets 60 may be designed to provide therapeutic electrical stimulation and one or more other stimulation sets 60 may be designed to reduce neuroplasticity effects. In this case, the therapeutic stimulation sets 60 and neuroplasticity reducing stimulation sets 60 may be arranged temporally in any suitable manner. A therapeutic stimulation set 60 may be separated from a successive therapeutic stimulation set 60 by any number of neuroplasticity reducing stimulation sets 60 and this number may be the same between each pair of therapeutic stimulation sets 60 or may vary between each pair of therapeutic stimulation sets 60 in a predetermined or randomized manner. As another example, one or more stimulation sets 60 may be designed to concurrently provide both therapeutic and neuroplasticity reducing electrical stimulation.

In addition, the amplitude or intensity, frequency, phase information, or pulse width for a stimulation set 60 may be particular to the stimulation being provided. For example, therapeutic electrical stimulation may be provided using higher amplitude electrical energy than is used for neuroplasticity reducing electrical stimulation. In this case, the neuroplasticity reducing electrical stimulation may be below the therapeutic target threshold stimulation (i.e. below the threshold where therapeutic electrical stimulation is provided to adjust the level of activity in the target nerve tissue in the person's brain to treat the condition in the person's body). Alternatively, neuroplasticity reducing electrical stimulation may be provided using the same or a higher amplitude electrical energy than is used for therapeutic electrical stimulation (i.e. at or above the threshold where therapeutic electrical stimulation is provided to adjust the level of activity in the target nerve tissue in the person's brain to treat the condition in the person's body). In this case, the neuroplasticity reducing electrical stimulation's primary purpose is not to produce a therapeutic effect, but rather to reduce neuroplasticity. In this manner, the neuroplasticity reducing electrical stimulation could have both a therapeutic and neuroplasticity reducing effect.

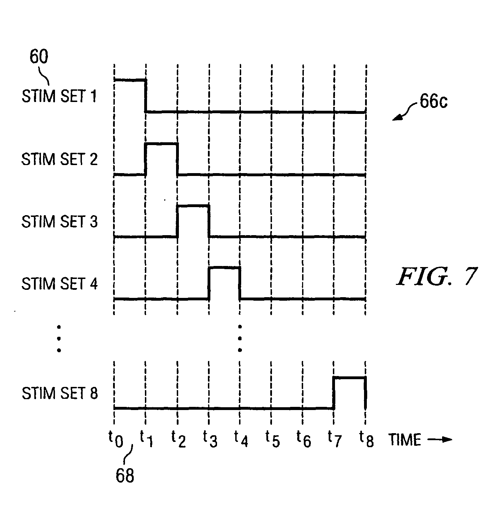

FIGURE 6 illustrates a number of example stimulation programs 66, each including a number of stimulation sets 60. One or more simulation programs 66 may be set up to reduce neuroplasticity effects associated with therapeutic electrical stimulation of the brain. As described above, each stimulation set 60 specifies a number of stimulation parameters for the stimulation set 60. In one embodiment, within each stimulation program 66, stimulation system 10 consecutively executes the sequence of one or more stimulation sets 60 associated with stimulation program 66. The sequence may be executed only once, repeated a specified number of times, or repeated an unspecified number of times within a specified time period. For example, as is illustrated in FIGURE 7 for the third example stimulation program 66c including eight stimulation sets 60, each of the eight stimulation sets 60 is consecutively executed in sequence. Although the time intervals 68 (t₁-t₀, t₂-t₁, etc.) during which the stimulation sets 60 are executed are shown as being equal, the present invention contemplates a particular stimulation set 60 being executed over a different time interval 68 than one or more other stimulation sets 60 according to particular needs. One or more stimulation sets 60 within at least one stimulation program 66 may be set up to provide reduced neuroplasticity effects associated with therapeutic electrical stimulation of the brain.

Although stimulation system 10 is illustrated by way of example as accommodating up to twenty-four stimulation programs 66 each including up to eight stimulation sets 60, the present invention contemplates any appropriate number of stimulation programs 66 each including any appropriate number of stimulation sets 60. For example, in a very simple case, a single stimulation program 66 may include a single stimulation set 60, whereas in a very complex case more than twenty-four stimulation programs 66 may each include more than eight stimulation sets 60.

In one embodiment, stimulation system 10 executes only a single stimulation program 66 in response to user selection of that stimulation program for execution. In another embodiment, during a stimulation period, stimulation system 10 executes a sequence of pre-programmed stimulation programs 66 for each stimulation lead 14 until the stimulation period ends. Depending on the length of the stimulation period and the time required to execute a sequence of stimulation programs 66, the sequence may be executed one or more times. For example, the stimulation period may be defined in terms of a predetermined number of cycles each involving a single execution of the sequence of stimulation programs 66, the sequence of stimulation programs 66 being executed until the predetermined number of cycles has been completed. As another example, the stimulation period may be defined in terms of time, the sequence of stimulation programs 66 being executed until a predetermined time interval has elapsed or the patient or another user manually ends the stimulation period. Although a sequence of stimulation programs 66 is described, the present invention contemplates a single stimulation program being executed one or more times during a stimulation period according to particular needs. Furthermore, the present invention contemplates each stimulation program 66 being executed substantially immediately after execution of a previous stimulation program 66 or being executed after a suitable time interval has elapsed since completion of the previous stimulation program 66. Where stimulation system 10 includes multiple stimulation leads 14, stimulation programs 66 for a particular stimulation lead 14 may be executed substantially simultaneously as stimulation programs 66 for one or more other stimulation leads 14, may be alternated with stimulation programs 66 for one or more other stimulation leads 14, or may be arranged in any other suitable manner with respect to stimulation programs 66 for one or more other stimulation leads 14.

Where stimulation system 10 provides, in addition to therapeutic electrical stimulation, electrical stimulation to reduce neuroplasticity effects, each stimulation program 66 may be particular to either the therapeutic electrical stimulation or the neuroplasticity reducing electrical stimulation. For example, one or more stimulation programs 66 may be designed to provide therapeutic electrical stimulation and one or more other stimulation programs 66 may be designed to reduce neuroplasticity effects. In this case, the therapeutic stimulation programs 66 and the neuroplasticity reducing stimulation programs 66 may be arranged temporally in any manner. A therapeutic stimulation program 66 may be separated from a successive therapeutic stimulation program 66 by any number of neuroplasticity reducing stimulation programs 66 and this number may be the same between each pair of therapeutic stimulation programs 66 or may vary between each pair of therapeutic stimulation programs 66 in a predetermined or randomized manner. As another example, one or more stimulation programs 66 may be set up to concurrently provide both therapeutic and neuroplasticity reducing electrical stimulation.

In general, each stimulation program 66 may, but need not necessarily, be set up for electrical stimulation of different target nerve tissue in a person's brain. As an example, where therapeutic electrical stimulation of target nerve tissue in a particular region of the brain is desired, one or more stimulation programs 66 may be set up for therapeutic electrical stimulation of the target nerve tissue in the particular region and one or more other stimulation programs 66 may be set up for electrical stimulation of the same target nerve tissue in the particular region to reduce neuroplasticity effects associated with the therapeutic electrical stimulation. As another example, one or more stimulation programs 66 may be set up for therapeutic electrical stimulation of target nerve tissue in a particular region of the brain and one or more other stimulation programs 66 may be set up for electrical stimulation of different nerve tissue in either the same region or in a different region of the brain to reduce neuroplasticity effects associated with the therapeutic electrical stimulation.

As described above, in one embodiment, the nature of any neuroplasticity reducing electrical stimulation may be varied more or less continually, whether in a predetermined or randomized manner, to reduce, prevent, delay, enhance, promote, or otherwise control the ability of the brain to adapt to the neuroplasticity reducing electrical stimulation and dynamically reorganize itself accordingly. In a more particular embodiment, where the neuroplasticity reducing electrical stimulation is provided concurrently with therapeutic electrical stimulation, the neuroplasticity reducing electrical stimulation may be randomized or otherwise varied about the therapeutic electrical stimulation to achieve this result. In essence, the randomized or otherwise varied neuroplasticity reducing electrical stimulation makes it more difficult for the brain to dynamically reorganize itself to overcome the effects of the therapeutic electrical stimulation.

The present invention contemplates any suitable circuitry within stimulation source 12 for generating and transmitting electrical stimulation pulses for electrically stimulating target nerve tissue in a person's brain, for DBS for example, and, where appropriate, to reduce, enhance, or otherwise modify neuroplasticity effects in the person's brain, whether separate from or concurrently with the therapeutic electrical stimulation. Example circuitry that may be used is illustrated and described in U.S. Patent 6,609,031 B1, which is hereby incorporated by reference herein as if fully illustrated and described herein.

Although the present invention has been described above in connection with several embodiments, a number of changes, substitutions, variations, alterations, transformations, and modifications may be suggested to one skilled in the art, and it is intended that the present invention encompass such changes, substitutions, variations, alterations, transformations, and modifications as fall within the spirit and scope of the appended claims.

## Claims

1. An electrical stimulation system for electrically stimulating target nerve tissue in a person's brain using a stimulation lead having a tip electrode, comprising:
a stimulation lead adapted for implantation into the brain for electrical stimulation of target nerve tissue in the brain, the stimulation lead comprising a tip electrode located at a distal tip of the stimulation lead and adapted to be positioned proximate the target nerve tissue in the brain and to deliver electrical stimulation pulses to the target nerve tissue in the brain; and
a stimulation source operable to generate the electrical stimulation pulses for transmission to the tip electrode located at the distal tip of the stimulation lead to cause the tip electrode to deliver the electrical stimulation pulses to the target nerve tissue in the brain.

2. The system of claim 1, or claim 12, wherein the tip electrode comprises a substantially spherical distal surface.

3. The system of claim 1, or claim 12, or claim 2 as it depends from claim 1 or claim 12, wherein the tip electrode is adapted to emit a substantially monopolar field in delivering electrical stimulation pulses to the target nerve tissue in the brain.

4. The system of any preceding claim, or claim 12, wherein the tip electrode is adapted to operate as a cathode and the stimulation source is adapted to operate as an anode.

5. The system of any preceding claim, or claim 12, wherein the tip electrode is approximately 1.40 mm in diameter.

6. The system of any preceding claim, wherein the stimulation lead further comprises one or more additional circumferential electrodes spaced apart from the tip electrode and from one another along the stimulation lead, the one or more additional circumferential electrodes adapted to be positioned proximate and deliver electrical stimulation pulses to additional target nerve tissue in the brain.

7. The system of claim 6, wherein the tip electrode is adapted to be controlled independently from the one or more additional circumferential electrodes.

8. The system of any preceding claim, or claim 10, wherein delivering electrical stimulation pulses to the target nerve tissue in the brain comprises delivering electrical stimulation pulses to deep brain tissue and the electrical stimulation system provides deep brain stimulation (DBS).

9. The system of any preceding claim, or claim 10, wherein the target nerve tissue in the brain comprises nerve tissue associated with at least one of the person's:
frontal lobe;
occipital lobe;
parietal lobe;
temporal lobe;
cerebellum; and
brain stem.

10. An electrical stimulation system for electrically stimulating target nerve tissue in a person's brain using a stimulation lead having at least five electrodes, comprising:
a stimulation lead adapted for implantation into the brain for electrical stimulation of target nerve tissue in the brain, the stimulation lead comprising at least five electrodes, the stimulation lead adapted to be implanted in the brain such that one or more of the at least five electrodes are positioned proximate the target nerve tissue in the brain to deliver electrical stimulation pulses to the target nerve tissue in the brain; and
a stimulation source operable to generate the electrical stimulation pulses for transmission to the one or more of the at least five electrodes of the stimulation lead positioned proximate the target nerve tissue in the brain to cause the one or more of the at least five electrodes to deliver the electrical stimulation pulses to the target nerve tissue in the brain.

11. The system of claim 10, wherein the at least five electrodes comprise at least five circumferential electrodes.

12. The system of claim 10, wherein the at least five electrodes comprise:
a tip electrode located at a distal tip of the stimulation lead; and
at least four circumferential electrodes spaced apart from the tip electrode and from one another along the stimulation lead.

13. The system of claim 12 or any claim dependent directly or indirectly from claim 12, wherein the tip electrode is adapted to be controlled independently from the at least four circumferential electrodes.

14. An electrical stimulation system for electrically stimulating target nerve tissue in a person's brain using a stimulation lead having at least five electrodes, comprising:
a stimulation lead adapted for implantation into the brain for electrical stimulation of target nerve tissue in the brain, the stimulation lead comprising at least five electrodes, the stimulation lead adapted to be implanted in the brain such that one or more of the at least five electrodes are positioned proximate the target nerve tissue in the brain to deliver electrical stimulation pulses to the target nerve tissue in the brain, the at least five electrodes comprising:
a tip electrode located at a distal tip of the stimulation lead, having a substantially spherical distal surface, and adapted to emit a substantially monopolar field in delivering electrical stimulation pulses to the target nerve tissue in the brain; and
at least four circumferential electrodes spaced apart from the tip electrode and from one another along the stimulation lead;
the tip electrode adapted to be controlled independently from the at least four circumferential electrodes; and
a stimulation source operable to generate the electrical stimulation pulses for transmission to the one or more of the at least five electrodes of the stimulation lead positioned proximate the target nerve tissue in the brain to cause the one or more of the at least five electrodes to deliver the electrical stimulation pulses to the target nerve tissue in the brain.
